(19) Europäisches Patentamt
European Patent Office
Office européen des brevets

(11) Numéro de publication : **0 566 495 A1**

(12) **DEMANDE DE BREVET EUROPEEN**

(21) Numéro de dépôt : **93400987.9**

(22) Date de dépôt : **15.04.93**

(51) Int. Cl.[5] : **A01N 53/00,** A01N 37/24, A61K 7/075, C09D 5/22

(30) Priorité : **16.04.92 FR 9204691**

(43) Date de publication de la demande : **20.10.93 Bulletin 93/42**

(84) Etats contractants désignés : **AT BE CH DE ES GB GR IT LI LU MC PT**

(71) Demandeur : **PIERRE FABRE SANTE**
**45, Place Abel-Gance**
**F-92100 Boulogne (FR)**

(72) Inventeur : **Fabre Pierre**
**1 Avenue d' Albi**
**F-81100 Castres (FR)**
Inventeur : **Mouzin Gilbert**
**54 rue d' Austerlitz**
**F-81100 Castres (FR)**
Inventeur : **Jeanjean Michel**
**11 Chemin Savignol**
**F-31220 Castanet-Tolosan (FR)**
Inventeur : **Cousse Henri**
**La Foun de la Nobios**
**F-81100 Castres (FR)**
Inventeur : **Monteny Nicole**
**5 Avenue des Pinsons**
**F-91400 Orsay (FR)**

(74) Mandataire : **Ahner, Francis**
**CABINET REGIMBEAU, 26, avenue Kléber**
**F-75116 Paris (FR)**

(54) **Potentialisation de pyréthrinoide par le crotamiton utile dans le traitement de la pédiculose.**

(57) La présente invention concerne une composition dermatologique et/ou cosmétologique utile dans le traitement de la pédiculose, caractérisée en ce qu'elle contient une association synergique d'un pyréthrinoïde et du crotamiton.

Les pyréthrinoïdes objet de cette composition sont plus particulièrement la perméthrine, la bioalléthrine, la resméthrine, la tétraméthrine et la deltaméthrine.

EP 0 566 495 A1

La présente invention a pour objet une association synergique de pyréthrinoïdes et de crotamiton et leur formulation utile pour la destruction d'ectoparasites tels que les poux et leurs lentes.

Les pédiculicides actuellement commercialisés présentent des phénomènes de résistance sur les poux et plus particulièrement sur les lentes. En 1945, la mise au point des poudres insecticides à base de DDT a fait l'objet de la première campagne contre les poux. Ce n'est qu'en 1952 que les premiers signes de résistance du pou du corps au DDT sont apparus en Corée (HURLBUT et Coll, Science, 115, 11.12.1952).

Le pou est devenu également résistant au carbaryl (CLARK et Coll, J. Econ Ent, 60, 2, 398, 1967) et au malathion (COLE., J. Econ. Ent., 66, 1 p.118, 1973).

Le pou de tête est décrit comme résistant au DDT en France dès 1976 (LAMIZANA, Med. Mal. Infectieuses 6.2 p.48 1976). D'après COMBESCOT et COZ, ce phénomène de résistance s'étendrait actuellement aux dérivés de la classe des pyréthrinoïdes qui sont les produits les plus utilisés comme pédiculicides.

L'emploi simultané de deux ou plusieurs pédiculicides, à mode d'action physiologique différent, devrait constituer un traitement qui aurait de fortes probabilités d'éviter les possibilités de sélection de population résistante.

L'objet de la présente invention concerne une composition dermatologique et/ou cosmétologique utile pour le traitement de la pédiculose comprenant une association synergique d'au moins un pyréthrinoïde avec du crotamiton.

La quantité pondérale de crotamiton est avantageusement 10 à 100 fois supérieure à celle du pyréthrinoïde, ou du mélange de pyréthrinoïde.

Les principaux pyréthrinoïdes sont

- bioalléthrine
- resméthrine
- tétraméthrine
- D. phénothrine

et deltaméthrine.

D'une manière préférentielle, le pyréthrinoïde est choisi parmi la perméthrine, la bioalléthrine, la tétraméthrine, la deltaméthrine et leurs mélanges.

La composition selon la présente invention contient de préférence de 0,01 à 1% en poids de pyréthrinoïde. Elle contient également avantageusement, entre 0,1 et 10% en poids de crotamiton.

D'une manière préférentielle, la composition dermatologique et/ou cosmétologique selon la présente invention comprend :

a) 0,01 à 1% en poids d'un pyréthrinoïde choisi parmi la bioallétrine, la resméthrine, la tétraméthrine, la D.phénotrhine, la perméthrine, la deltaméthrine et leurs mélanges,

b) 0,1 à 10% en poids de crotamiton,

c) 0 à 70% en poids d'un agent tensio-actif,

d) 0 à 50% en poids d'un solvant organique choisi parmi les alcools alphatiques inférieurs, les silicones, le pétrole désodorisé et leurs mélanges,

e) 0 à 4% en poids d'un agent moussant,

f) un parfum,

g) un conservateur, et

le complément à 100% en eau.

L'agent tensio-actif est choisi parmi les agents tensio-actifs anioniques, non-ioniques, cationiques, amphotères et leurs mélanges.

A titre d'exemple non limitatif, nous décrivons l'étude de l'association perméthrine et crotamiton qui présente une potentialisation tout à fait surprenante de l'action lenticide . Ces travaux ont été effectués dans le Laboratoire de Lutte contre les Insectes Nuisibles (L.I.N.) du Centre ORSTOM à Bondy sous la responsabilité du Dr MONTENY.

## 1. Méthode

Les expériences sont effectuées sur des lentes de Pediculus humanus provenant d'une souche élevée sur des lapins de race "Nouvelle Zélande".

Les lentes, âgées de 2 à 5 joues, fixées à des supports de tissu, sont plongées dans des dilutions successives de produits effectuées dans une solution contenant 5 % d'acétone, 45 % d'alcool absolu et 50 % d'eau. Les concentrations exprimées dans cette étude sont calculées en volume de produit brut/volume de solvant.

Les lentes, comptées sur leur support de tissu, sont laissées en contact trois minutes, rincées à l'eau trois fois, séchées, puis placées à l'étuve à 28° C et 70 % d'humidité relative.

Chaque jour, les lentes sont placées sur le ventre rasé d'un lapin et les éclosions sont notées. Les éclosions

sont échelonnées. Le joue de lecture est celui où l'on observe le maximum d'éclosion ; le nombre de jeunes poux vivants retenu correspond au nombre de ceux qui se gorgent. A ce stade, le nombre de lentes ouvertes ( écloses) est relevé.

## 2. Détermination des courbes mortalité/concentration

### 2.1 Permethrine

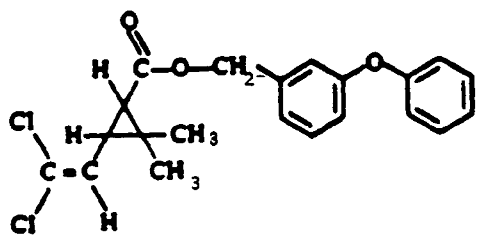

La Permetheine est utilisée comme pesticide en agriculture et comme insecticide en médecine vétérinaire, elle a été approuvée par la FDA en 1986 pour le traitement local de la pédiculose du cuir chevelu (Nix crème Rinse Burroughs Wellcom USA dosage 1 %). En France, la Permethrine est commercialisée depuis 1978 (Pyreflor - Lab. Clément).

La Permethrine détruit à la fois les poux et les lentes, elle agit sur le système nerveux des insectes mais son mécanisme d'action n'est pas complètement élucidé. Chez les mammifères, cet actif est peu absorbé et inactivé par les estérases.

#### 2.1.1. Résultats

Dans les tableaux I et II sont portés les pourcentages d'éclosions obtenus avec la Permethrine et les témoins. Le test a été refait pour obtenir une base fiable et répétitive pour objectiver la réelle potentialisation de l'association dans la suite de l'étude.

Les pourcentages d'éclosions sont notés E % et les pourcentages d'insectes qui se nourrissent N % (entre parenthèses l'effectif).

| | | | Dilution (%) | | | | |
|---|---|---|---|---|---|---|---|
| | Témoin sec | Témoin solvant | 0,5 | 0,25 | 0,125 | 0,0625 | 0,0312 |
| E % | 95,2 (104) | 87,2 (94) | 1,96 (153) | 12,25 (155) | 39,56 (91) | 58,26 (115) | 77,48 (111) |
| N % | 94,23 | 82,98 | 0,0 | 7,1 | 30,77 | 45,22 | 73,87 |

Tableau I : Activité du PM 5 en solution acétono-alcoolique
Oeufs du 2 au 5 août 1991
Test du 7 août 1991.

| | Témoin sec | Témoin solvant | Dilution (%) 0.5 | 0,25 | 0,125 | 0,0625 | 0,0312 |
|---|---|---|---|---|---|---|---|
| E % | 83,1 (83) | 83,1 (71) | 0,0 (80) | 9,9 (101) | 42,6 (122) | 53,1 (96) | 86,1 (72) |
| N % | 80,72 | 76,06 | 0,0 | 6,93 | 31,15 | 45,83 | 69,44 |

Tableau II : Activité du PM 5 en solution acétono-alcoolique
Oeufs du 16 au 19 août 1991
Test du 21 août 1991.

2.1.2. Détermination des concentrations létales

Les CL 5, 10, 30, 30, 40 et 50 ont été déterminées globalement en sommant les deux séries de résultats grâce à un programme d'analyse Probit de mortalité.

**Programme ANALYSE PROBITS**

PM 5 (Permethrine)

Les concentrations sont en: %
Nombre d'insectes témoins : 165
Mortalité chez le témoin : 21

| POINTS | CONCENTRATIONS | MORTALITE BRUTE EN % | MORTALITE CORRIGEE | PROBITS CALCULES |
|---|---|---|---|---|
| 1 | 0,5 | 100,0 | 100,0 | 7,59 |
| 2 | 0,25 | 92,97 | 92,0 | 6,41 |
| 3 | 0,125 | 69,01 | 64,9 | 5,37 |
| 4 | 0,0625 | 54,5 | 48,4 | 4,96 |
| 5 | 0,0312 | 27,87 | 18,3 | 4,09 |

La CL 5 est égale à: 0,015 %
Intervalle de confiance : 0,012-0,019
La CL 10 est égale à: 0,022 %
Intervalle de confiance : 0,018-0,026
La CL 20 est égale à: 0,035 %
Intervalle de confiance : 0,03-0,039
La CL 30 est égale à: 0,046 %
Intervalle de confiance : 0,041-0,051
La CL 40 est jale à: 0,06 %
Intervalle de confiance : 0,055-0,066
La CL 50 est égale à: 0,072 %
Intervalle de confiance : 0,066-0,078
Equation de la droite de régression pondérée : Y = 2,55 X+ 2,81
Le x2 correspondant à une probabilité de 5 % est de: 7,815
Le x2 est de 13,167. Il est donc significatif.

2.2 Crotamiton

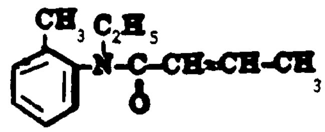

Le Crotamiton est commercialisé en FRANCE depuis 1949 sous le nom de marque EURAX par la société CIBA GEIGY avec comme indication : antiprurigineux. Aux USA, la société WESWOOD le commercialise également comme antiprurigineux mais aussi dans l'indication scabicide. Le mécanisme d'action de cet actif n'est pas connu.

2.2.1 Résultats

Dans les tableau III, IV et V, sont portés les pourcentages d'éclosions obtenus avec le produit CR 3 et les témoins.

Les pourcentages d'éclosions sont notés E %, et les pourcentages d'insectes qui se nourrissent N % (entre parenthèses l'effectif).

| | | Dilution (%) | | | | |
|---|---|---|---|---|---|---|
| | Témoin solvant | 3 | 2 | 1 | 0,5 | 0,25 |
| E % | 63,9 (119) | 1,6 (122) | 45,0 (131) | 61,4 (101) | 65,8 (111) | 34,8 (89) |
| N % | 58,82 | 1,64 | 39,69 | 59,41 | 58,56 | 32,58 |

Tableau III : Activité du CR 3 en solution acétono-alcoolique
Oeufs du 12 au 15 juillet 1991
Test du 17 juillet 1991

| | | | Dilution (%) | | | | |
|---|---|---|---|---|---|---|---|
| | Témoin sec | Témoin solvant | 3 | 2,5 | 2 | 1,5 | 1 |
| E % | 97,0 (68) | 74,4 (82) | 0,99 (101) | 52,0 (123) | 71,4 (119) | 74,0 (69) | 75,0 (76) |
| N % | 91,18 | 67,07 | 0,0 | 23,58 | 66,39 | 62,32 | 68,42 |

Tableau IV : Activité du CR 3 en solution acétono-alcoolique
Oeufs du 2 au 5 août 1991
Test du 7 août 1991.

| | Témoin sec | Témoin solvant | Dilution (%) 3 | 2,5 | 2 | 1,5 | 1 |
|---|---|---|---|---|---|---|---|
| E % | 91,7 (72) | 73,8 (126) | 0,88 (113) | 2,6 (114) | 35,8 (120) | 48,0 (77) | 71,8 (78) |
| N % | 88,89 | 70,63 | 0,0 | 1,75 | 25,83 | 40,26 | 62,82 |

Tableau V : Activité du CR 3 en solution acétono-alcoolique
Oeufs du 16 au 19 août 1991
Test du 21 août 1991.

Remarque : Les trois séries de résultats sont répétitives.
Elles sont toutes prises en compte pour le calcul des concentrations létales.

2.2.2. Détermination des concentrations létales

Les CL 5, 10, 20, 30, 40 et 50 ont été déterminées globalement en sommant les trois séries de résultats grâce à un programme d'analyse Probit de mortalité.

**Programme ANALYSE PROBITS**

CR 3 (CROTAMITON)

Les concentrations sont en: %
Nombre d'insectes témoins: 327
Mortalité chez le témoin : 31

| POINTS | CONCENTRATIONS | MORTALITE BRUTE EN % | MORTALITE CORRIGEE | PROBITS CALCULES |
|---|---|---|---|---|
| 1 | 3,0 | 99,4 | 99,3 | 6,38 |
| 2 | 2,5 | 98,25 | 98,1 | 6,28 |
| 3 | 2,0 | 70,05 | 66,7 | 5,43 |
| 4 | 1,5 | 59,74 | 55,3 | 5,13 |
| 5 | 1,0 | 36,91 | 29,9 | 4,49 |

La CL 5 est égale à: 0,534 %
Intervalle de confiance : 0,46-0,604
La CL 10 est égale à: 0,668 %
Intervalle de confiance : 0,589-0,74
La CL 20 est égale à: 0,882 %
Intervalle de confiance: 0,802-0,955
La CL 30 est égale à: 1,043 %
Intervalle de confiance : 0,964-1,114
La CL 40 est égale à: 1,233 %
Intervalle de confiance : 1,158-1,302
La CL 50 est égale à: 1,378 %
Intervalle de confiance : 1,306-1,446
Equation de la droite de régression pondérée: Y = 4,13 X-3,84
Le x2 correspondant à une probabilité de 5 % est de : 7,815
Le x2 est de 15,774. Il est donc significatif.

2.3 Association PERMETHRINE-CROTAMITON

Dans le tableau VI sont portés les pourcentages d'éclosions obtenus avec le produit PM 5 à la CL 5, additionné de produit CR 3 aux CL 5, 10, 20 et 30 et les témoins.

Les pourcentages d'éclosions sont notés E % et les pourcentages d'insectes qui se nourrissent N % (entre parenthèses l'effectif).

| | | | PM 5 CL 5 | | | |
|---|---|---|---|---|---|---|
| | Témoin sec | Témoin solvant | CR 3 CL 30 | CR 3 CL 20 | CR 3 CL 10 | CR 3 CL 5 |
| E % | 82,42 (91) | 79,78 (89) | 0,0 (124) | 1,11 (90) | 2,97 (101) | 3,06 (98) |
| N % | 58,24 | 69,66 | 0,0 | 0,0 | 0,0 | 0,0 |

Tableau VI : Activité du PM 5 à la CL 5 en solution acétono-alcoolique, additionné de CR 3 aux CL 5, 10, 20, 30 et 40
Oeufs du 15 au 18 novembre 1991
Test du 20 novembre 1991.

Dans le tableau VII sont portés les pourcentages d'éclosions obtenus avec le Produit CR 3 à la CL 5, additionné de produit PM 5 aux CL 5, 10, 20 et 30.

Les pourcentages d'éclosions sont notés E % et les pourcentages d'insectes qui se nourrissent N % (entre parenthèses l'effectif).

| | CR 3 CL 5 | | | |
|---|---|---|---|---|
| | PM 5 CL 30 | PM 5 CL 20 | PM 5 CL 10 | PM 5 CL 5 |
| E % | 0,0 (125) | 1,74 (115) | 0,79 (126) | 3,06 (98) |
| N % | 0,0 | 0,0 | 0,0 | 0,0 |

Tableau VII : Activité du CR 3 à la CL 5 en solution acétono-alcoolique, additionné de PM 5 aux CL 5, 10, 20 et 30
Oeufs du 15 au 18 novembre 1991
Test du 20 novembre 1991.

2.3.1. Résultats

L'étude réalisée au LIN a eu pour but :

a) de déterminer avec précision les conditions de reproductibilité du dosage de l'effet de la PERMETHRINE et du CROTAMITON sur Pediculus humanus.

b) à partir de la relation concentration-mortalité pour chacun des deux produits, de définir la composition optimale d'un mélange, avec recherche d'un effet synergique.

Il est apparu, au cours des essais préliminaires que la valeur la plus reproductible, parmi les paramètres biologiques dont on dispose à priori était le pourcentage de jeunes poux qui se nourrissent après éclosion par rapport au nombre de lentes introduites dans le test.

Des essais successifs ont tenté de définir la composition optimale du mélange en combinant des concentrations conduisant à des mortalités partielles. Si la mortalité résultante est supérieure à la somme des deux

mortalités partielles, un effet synergique peut être attendu.

Cette étude a montré d'une façon surprenante, une très forte potentialisation de ces deux molécules. Une mortalité de 100 % est atteinte en associant le CROTAMITON et la PERMETHRINE à une concentration censée donner 5 % de mortalité pour chacun des produits. Nous obtenons donc une formulation possédant un effet synergique de plus de dix fois le résultat attendu par additivité d'action (soit 5 % + 5 % = 10 %).

Le coefficient de potentialisation par le CROTAMITON a été déterminé d'une façon précise à partir du rapport des valeurs des doses létales 50.

La dose létale 50 de la PERMETHRINE est de 0,072 %.

La dose létale 50 du mélange CROTAMITON-PERMETHRINE est de 4,228 %, ceci correspond à une concentration réelle de la PERMETHRINE de 0,063. $10^{-2}$ %. Le coefficient de potentialisation de la PERMETHRINE par le CROTAMITON est donc:

$$\frac{0{,}072}{0{,}063\ 10^{-2}} = 113.$$

De plus, ces deux molécules possédant un mécanisme d'action différent, cette nouvelle association doit permettre d'éviter les phénomènes de résistance aux pédiculicides.

## 3. Galénique et clinique

Les nouvelles préparations pédiculicides selon la présente invention permettent d'obtenir une nouvelle génération de spécialités pharmaceutiques présentant les avantages suivants :

- Activité performante sur les poux et les lentes
- Eviter les phénomènes de résistance classique
- Meilleur rapport efficacité/toxicité
- Activité complémentaire intéressante du CROTAMITON (antiprurigineux)
- possibilité d'enregistrement international (Actifs F.D.A.)

Les tests cliniques effectués sur ces nouvelles préparations confirment les excellents résultats obtenus au laboratoire.

Les formulations suivantes sont mentionnées à titre indicatif :

### 3.1 - Mousse aérosol

| | | |
|---|---|---|
| Crotamiton | 1 à 2 | g |
| Permethrine | 0,01 à 0,2 | g |
| CTAB (au stéaralkonium chloride) | 0,50 | g |
| Gomme guar quaternisée | 0,15 | g |
| PVA/VA (70/30) | 2 | g |
| PVP/Diméthyl aminoéthyl-méthacrylate | 0,50 | g |
| Alcool 95° | 15 à 20 | ml |
| Alcool gras éthoxylé (20 à 30 EO) | 1 | g |
| Parfum | q.s. | |
| Eau déminéralisée q.s.p. | 100 | g |
| Remplissage pour 100 g : | | |
| Base émulsionnée | 90 | g |
| Mélange butane/isobutane/propane | 10 | g |

3.2. - Shampooing fluide

| | | |
|---|---|---|
| Crotamiton | 0,1 à 2 | g |
| BIOALLETHRINE | 0,01 à 0,2 | g |
| Alkyl éther sulfate de magnésium (30 %) | 20 | g |
| Monolaurate de sorbitan (2 EO) | 15 | g |
| Cocamido propyl bétaïne (30 %) | 5 | g |
| Diéthanolamide de Coprah | 4 | g |
| Conservateur | q.s. | |
| Parfum | q.s. | |
| Eau déminéralisée q.s.p. | 100 | ml |

3.3 - Aérosol

| | | |
|---|---|---|
| Crotamiton | 0,1 à 2 | g |
| RESMETHRINE | 0,01 à 0,2 | g |
| Huile de silicone haute volatilité | 15 | g |
| Pétrole désodorisé | 15 | g |
| Essence de myrthe | 0,30 | g |
| Gaz propulseur butane 3,2 q.s.p. | 100 | ml |
| ou | | |
| Gaz propulseur comprimé ($N_2$) q.s. | | |

3.4 - Shampooing biphasique

| | | |
|---|---|---|
| Crotamiton | 0,1 à 2 | g |
| Tétramethrine | 0,01 à 0,2 | g |
| Palmitate d'isopropyle | 3 | g |
| Silicone cyclique volatil | 5 | g |
| Essence de myrthe | 0,30 | g |
| Cocoamphocarboxyglycinate (40 %) | 19 | g |
| Alkyl éther sulfate de sodium (30 %) | 26 | g |
| Polymère de polyglycol polyamine | 0,50 | g |
| Acide citrique q.s. pH | 7 | g |
| Eau déminéralisée q.s.P. | 100 | ml |

3.5. - Lotion capillaire antipoux 2 phases

Solution ou vaporisateur pompe

| | | |
|---|---|---|
| Crotamiton | 0,1 à 2 | g |
| Deltaméthrine | 0,01 à 0,2 | g |
| Hexaméthyl disiloxane | 9 | ml |
| Essence de myrthe | 0,50 | g |
| Chlorure de lauryl pyridinium | 0,015 | g |
| Diméthicone copolyol | 0,15 | g |
| Allantoïne | 0,05 | g |
| N-hydroxyéthyl acétamide (70 %) | 1 | g |
| D-Panthénol | 0,02 | g |
| Alcool 95° | 35 | ml |
| Eau déminéralisée q.s.p. | 100 | ml |

3.6 Baume démélant lenticide

| | | |
|---|---|---|
| Crotamiton | 0,1 à 2 | g |
| Permethrine | 0,01 à 0,2 | g |
| Stearamine oxyde | 7,5 | g |
| Hcl/Kcl | q.s. | |
| Dimethicone copolyol | 0,75 | g |
| Hydroxypropyl cellulose | 0,30 | g |
| Stearalkonium chlorure | 0,25 | g |
| Huile minérale | 2,5 | g |
| Eau q.s.p | 100 | g |

**Revendications**

1. Composition dermatologique et/ou cosmétologique utile pour le traitement de la pédiculose, caractérisée en ce qu'elle contient une association synergique d'au moins un pyréthrinoïde et du crotamiton.

2. Composition selon la revendication 1, caractérisée en ce que la quantité pondérale de crotamiton est 10 à 100 fois supérieure à celle du pyréthrinoïde, ou du mélange de pyréthrinoïde.

3. Composition selon l'une des revendications 1 et 2, caractérisée en ce que le pyréthrinoïde est choisi parmi la perméthrine, la bioalléthrine, la tétraméthrine, la deltaméthrine et leurs mélanges.

4. Composition selon l'une des revendications 1 à 3, caractérisée en ce qu'elle contient de 0,01 à 0,2% en

poids de pyréthrinoïde.

5. Composition selon l'une des revendications 1 à 4, caractérisée en ce qu'elle contient de 0,1 à 2% en poids de crotamiton.

6. Composition dermatologique et/ou cosmétologique utile dans le traitement de la pédiculose selon la revendication 1, caractérisée en ce qu'elle comprend :
   a) 0,01 à 1% en poids d'un pyréthrinoïde choisi parmi la bioallétrine, la resméthrine, la tétraméthrine, la D.phénotrhine, la perméthrine, la deltaméthrine et leurs mélanges,
   b) 0,1 à 10% en poids de crotamiton,
   c) 0 à 70% en poids d'un agent tensio-actif,
   d) 0 à 50% en poids d'un solvant organique choisi parmi les alcools alphatiques inférieurs, les silicones, le pétrole désodorisé et leurs mélanges,
   e) 0 à 4% en poids d'un agent moussant,
   f) un parfum,
   g) un conservateur, et

   le complément à 100% en eau.

Office européen des brevets

# RAPPORT DE RECHERCHE EUROPEENNE

Numero de la demande

EP 93 40 0987
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | BIOLOGICAL ABSTRACTS vol. 93, no. 4 , 15 Février 1992, Philadelphia, PA, US; abstract no. 38025, R.S.PURVIS ET AL 'An outbreak of lindane-resistant scabies treated successfully with permethrin 5 percent cream' * abrégé * & J AM ACAD DERMATOL vol. 25, no. 6/1, 1991, pages 1015 - 1016 --- | 1-6 | A01N53/00, A01N37/24 A61K7/075 |
| A | BIOLOGICAL ABSTRACTS vol. 93, no. 10 , 15 Mai 1992, Philadelphia, PA, US; abstract no. 117282, R.HATSUSHIKA ET AL. 'Case studies on sting dermatitis by bethylid wasp, Cephalonomia gallicola (Ashmead, 1887) (Hymenoptera: Bethylidae) found in Okayama, Japan' * abrégé * & KAWASAKI MED J vol. 16, no. 2/3, 1990, pages 133 - 140 --- | 1-6 | DOMAINES TECHNIQUES RECHERCHES (Int. Cl.5 ) |
| A | CHEMICAL PATENTS INDEX, DOCUMENTATION ABSTRACTS JOURNAL Section Ch, Week 8527, 28 Août 1985 Derwent Publications Ltd., London, GB; Class C, AN 85-159610/27 & DD-A-219 374 (AKAD LANDWIRTSCHAFT) 6 Mars 1985 * abrégé * --- | 1-6 | A01N A61K |
| A | GB-A-638 662 (J.R.GEIGY) * page 1, colonne 2, ligne 71 - ligne 72 * * page 2, colonne 2, ligne 124 - page 3, colonne 1, ligne 11 * --- -/-- | 1-6 | |

Le présent rapport a été établi pour toutes les revendications

| Lieu de la recherche | Date d'achèvement de la recherche | Examinateur |
|---|---|---|
| LA HAYE | 09 JUIN 1993 | LAMERS W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 93 40 0987
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | WO-A-9 115 953 (THE PROCTER & GAMBLE)<br>* page 27, ligne 24 - page 33, ligne 34 *<br>----- | 1-6 | |

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| LA HAYE | 09 JUIN 1993 | LAMERS W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)